# EUROPÄISCHE PATENTSCHRIFT

(11) **EP 1 830 849 B1**
(45) Veröffentlichungstag und Bekanntmachung des Hinweises auf die Patenterteilung: **11.11.2009**
(21) Anmeldenummer: 05850456.4
(22) Anmeldetag: 14.12.2005
(51) Int. Cl.: A61K 31/43, A61K 31/7048, A61K 31/7052, A61K 31/545, A61K 31/546, A61K 9/50, A61K 9/16

(54) **SCHNELL FREISETZENDE DARREICHUNGSFORMEN FÜR ANTIBIOTIKA**
FAST RELEASE DOSAGE FORMS FOR ANTIBIOTICS
FORMES GALENIQUES A LIBERATION RAPIDE POUR ANTIBIOTIQUES

(30) Priorität: 23.12.2004 DE 102004063409; 08.09.2005 DE 102005042875
(43) Veröffentlichungstag der Anmeldung: 12.09.2007
(73) Patentinhaber: Grünenthal GmbH, 52078 Aachen (DE)
(72) Erfinder: ZIEGLER, Iris, Dr., 52159 Roetgen (DE); BARTHOLOMÄUS, Johannes, Dr., 52080 Aachen (DE); REDMER, Jessica, 41199 Mönchengladbach (DE)
(74) Vertreter: Brosch, Oliver
(86) Internationale Anmeldenummer: PCT/EP2005/056795
(87) Internationale Veröffentlichungsnummer: WO 2006/069920

(56) Entgegenhaltungen:
- EP-A- 0 276 781
- WO-A-03/079957
- WO-A-2004/000202
- WO-A-2004/000264
- US-A- 5 350 584
- US-A1- 2003 064 097

## Beschreibung

Die beanspruchte Erfindung betrifft eine multipartikuläre, pharmazeutische Darreichungsform, vorzugsweise in Form von Extrusionspellets enthaltend wenigstens ein mit wässrigen Medien schwer benetzbares und/oder in wässrigen Medien schwer lösliches Antibiotikum und eine Kombination aus Carrageenan und Tricalciumphosphat und ggf. wenigstens einen Sucroseester und ein Darreichungssystem umfassend diese Darreichungsform angeordnet in einem Trinkhalm, vorzugsweise zur einmaligen Verabreichung.

Gegen die meisten krankheitsverursachenden Bakterien wurden Antibiotika entwickelt, bei deren Einsatz aber oft Probleme auftreten können.

So weisen viele Antibiotika eine geringe Löslichkeit im wässrigen Medium auf, d. h. sie sind bei 37°C in wässrigen Medien mit einem pH von 1 bis 7,5, insbesondere in entsprechenden Standardpufferlösungen von ≤ 250 ml, in ihrer stärksten Dosierung nur schlecht oder überhaupt nicht löslich. Dabei ist es besonders ungünstig, wenn diese geringe Löslichkeit bei pH-Werten gegeben ist, die den physiologischen pH-Werten des Dünndarmmilieus entsprechen. Im Dünndarm sollte das Antibiotikum nämlich möglichst schnell zur Verfügung stehen, da dort die Resorption des Antibiotikums hauptsächlich stattfindet. Viele Antibiotika werden daher vorzugsweise als Lösung bzw. als Suspension verabreicht werden, um eine möglichst hohe Bioverfügbarkeit zu erzielen.

Weiterhin weisen zahlreiche Antibiotika oft auch einen besonders bitteren Geschmack auf, was dazu führt, dass solche Lösungen bzw. Suspensionen vom Patienten, insbesondere von Kindern, trotz Aromatisierung nur ungern eingenommen werden.

Die Folge ist, dass bei der üblicherweise über mehrere Tage dauernden Behandlung mit Antibiotika es immer schwieriger wird, den Patienten so zu motivieren, dass er die Therapie bis zum Ende durchführt. Die geringe Bereitschaft der Patienten zur Einnahme führt daher oft zu einem vorzeitigen Abbruch der Behandlung, was bei Antibiotika besonders schädliche Auswirkungen, wie eine Resistenzentwicklung, zur Folge haben kann.

Bei diesen Applikationsformen ist außerdem nachteilig, dass die Antibiotika üblicherweise als Trockensäfte auf den Markt gebracht werden, um die Produktion, den Transport und die Lagerung gegenüber fertigen Lösungen oder Suspensionen günstiger durchführen zu können. Erst vor der ersten Verabreichung wird die Trockensubstanz in eine Suspension übergeführt. Die zubereitete Suspension muß jedoch oftmals im Kühlschrank aufbewahrt und, wie vorstehend ausgeführt, über mehrere Tage eingenommen werden.

Dies bedeutet weitere Unannehmlichkeiten für den Patienten, da es während der Aufbewahrung der zubereiteten Suspension zu einer progressiven Geschmacksverschlechterung oder einer Agglomerierung der suspendierten Anteile bei längerer Standzeit kommen kann, wodurch die notwendige Redispergierung und damit die Dosisgenauigkeit beeinträchtigt wird. Außerdem beschränkt die oftmals notwendige Kühlung der Suspension eine problemlose Einnahme des Antibiotikum.

Um diese Nachteile zu vermeiden, können zur Zubereitung einer Suspension auch Brausetabletten verwendet werden, wobei eine Tablette üblicherweise einer Einzeldosis entspricht. So frisch zubereitete Lösungen oder Suspensionen verursachen gleichwohl einen bitteren Geschmack beim Patienten.

US2003/0064097 offenbart feste Arzneiformen für die Verabreichung von hydrophoben Wirkstoffen wie z.B. Antibiotika.

Probleme bereitet auch die Einnahme von trockenen Antiobiotika in Form von Granulaten, Pellets oder Mikrotabletten, abgefüllt in Sachets, die üblicherweise mit Hilfe einer Flüssigkeit geschluckt werden müssen. Insbesondere Kinder haben Probleme mit der Einnahme solcher Darreichungsformen. Dabei kann es auch bei nicht geschmacksneutralisierten Darreichungsformen passieren, dass bereits ein Teil des Antibiotikums innerhalb kurzer Zeit im Mund freigesetzt wird und einen unangenehmen Geschmack verursacht.

Bei einer solchen Einnahme von Trockenzubereitungen ist auch nicht sichergestellt, dass die gesamte notwendige Dosis geschluckt bzw. so schnell geschluckt wird, dass eine gegebenenfalls nur vorübergehende Geschmacksmaskierung effektiv genug ist.

Um solche Probleme zu vermeiden, wurden Darreichungsformen entwickelt, bei denen das Antibiotikum vorzugsweise in multipartikulärer Form in einem Trinkhalm angeordnet ist, aus dem es mit Hilfe einer Transportflüssigkeit von dem Patienten eingenommen wird. Bei dieser Art der Einnahme ist es besonders vorteilhaft, wenn die multipartikuläre Darreichungsform in Form gerundeter, sphärischer Pellets mit einer Partikelgröße nicht über 800 µm vorliegt, weil solche Teilchen von dem Patienten durch Ansaugen der Transportflüssigkeit mühelos transportiert und damit vollständig eingenommen werden können.

Dabei ist aber zu beachten, dass vorzugsweise unter Berücksichtigung des Schluckverhaltens von Kindern das Volumen und die Masse der Partikel möglichst gering zu halten ist, was eine hohe Wirkstoffbeladung der multipartikulären Darreichungsformen notwendig macht. Bekanntermaßen gelingt eine hohe Wirkstoffbeladung insbesondere mit Extrusionspellets, die außerdem die gewünschte glatte, vorzugsweise sphärische Form, ermöglichen. Nachteilig für diese Art der Darreichungsformen ist jedoch, dass sie aufgrund der bei den üblichen Herstellungsverfahrens notwendigerweise mitverwendeten Hilfsstoffen meist eine sehr kompakte Struktur aufweisen und daher in Wasser oder einem wässrigen Medium nicht oder höchstens sehr langsam bzw. nur teilweise zerfallen. Das kann bekannterweise zu einer retardierten Freisetzung des Wirkstoffes führen, insbesondere bei einem mit wässrigen Medien schwer benetzbaren und/oder in wässrigen Medien schwer löslichen Antibiotikums. Insbesondere gilt dies, wenn diese schwere Benetzbarkeit bzw. schwere Löslichkeit bei den physiologischen pH-Werten des oberen Dünndarmmilieus auftritt, wie vorstehend beschrieben. Infolge dessen kann es dann zu einer Freisetzung des Antibiotikums in beträchtlichem Ausmaß erst im unteren Darmabschnitt kommen.

Dies verhindert aber bei vielen Antibiotika eine ausreichende Bioverfügbarkeit, da deren Resorption hauptsächlich im oberen Dünndarmabschnitt gegeben ist. Diese geringe Auflösungsgeschwindigkeit der Darreichungsform und damit die verzögerte Freisetzung des Wirkstoffes wird insbesondere beim Einsatz der bekannten Sphäronisierungsmittel, wie mikrokristalliner Cellulose, niedrig substituierter Hydroxypropylcellulose, Hydroxypropylmethylcellulose beobachtet, die zwar zu Pellets in der gewünschten Form, nämlich sphärisch und mit glatter Oberfläche, und einer engen Teilchengrößenverteilung führen, aber eine verzögerte, diffusionsgesteuerte Freisetzung des Wirkstoffes verursachen. Insbesondere wenn das Antibiotikum in wässrigen Medien schwer löslich und/oder schwer benetzbar ist.

Dies wird in noch größerem Ausmaß auch bei den mit Hilfe der genannten Sphäronisierungsmittel hergestellten Extrusionspellets nach Auflösung eines vorhandenen Überzugs, der vorzugsweise Speichel resistent und/oder Magensaft resistent sein kann und vorzugsweise zur Geschmacksneutralisation dient, beobachtet. Selbst nach der pH- abhängigen Auflösung des Überzugs zerfallen die Pellets trotz Vorquellen oder Sprengmittelzusatz nicht oder sehr langsam, wodurch die Freisetzung des Antibiotikums verzögert und die Bioverfügbarkeit eingeschränkt wird.

Aufgabe der vorliegenden Erfindung war es daher, eine feste, multipartikuläre Darreichungsform eines mit wässrigen Medien schwer benetzbaren und/oder in wässrigen Medien schwer löslichen Antibiotikums zur Verfügung zu stellen, die insbesondere in einem wässrigen Medium mit einem pH-Wert entsprechend den physiologischen pH-Werten des Dünndarms eine rasche Auflösungsgeschwindigkeit zeigt und damit zu einer raschen Bioverfügbarkeit des Antibiotikums im sogenannten Resorptionsfenster führt.

Diese Aufgabe wird durch die Bereitstellung der erfindungsgemäßen, multipartikulären, pharmazeutischen Darreichungsform, vorzugsweise in Form von Extrusionspellets, enthaltend wenigstens ein mit wässrigen Medien schwer benetzbares und/oder in wässrigen Medien schwer lösliches Antibiotikum und eine Kombination aus Carrageenan und Tricalciumphosphat und ggf. wenigstens einen Succhroseester gelöst, wobei die Darreichungsform bei einem pH-Wert von 6-7 innerhalb von 30 Minuten mindestens 85 % des Antibiotikums freisetzt.

Diese rasche Auflösungsgeschwindigkeit und damit praktisch unretardierte Freisetzung des Wirkstoffes wird gemäß der nachstehend beschriebenen Methode veröffentlicht im "Guidance for Industry, Waiver of In Vivo Bioavailability and Bioequivalence Studies for Immediate-Release Solid Oral Dosage Forms based on a Biopharmaceutics Classification System., Seiten 1-3 / 7, Herausgeber von U.S. Department of Health and Human Services , Food and Drug administration, Center for Drug Evaluation and Research (CDER), August 2000, BP", bestimmt.

Diese hohe Auflösungsgeschwindigkeit wird insbesondere dann nicht erreicht, wenn man die bekannten unter Einsatz von mikrokristalliner Cellulose hergestellten Extrusionspellets magensaft resistent und/oder geschmacksneutral ausrüstet, da man nach Auflösen des Filmüberzugs einen weiter verzögerten Zerfall solcher Darreichungsformen feststellt, wodurch die Freisetzung im Dünndarm, im Vergleich zu entsprechenden, nicht überzogenen Pellets weiter verzögert ist und dadurch deren Bioverfügbarkeit beeinträchtigt ist.

Es ist daher um so erstaunlicher, dass die erfindungsgemäßen Darreichungsformen, selbst wenn sie mit einem Magensaft resistenten Überzug, z. B. zur Geschmackneutralisierung ausgerüstet sind, bei einem physiologischen pH-Wert von 6 -7 des Dünndarms, nach Auflösung eines solchen Überzuges, eine rasche Auflösung zeigen und damit innerhalb von 30 min zu einer Freisetzung von wenigstens 85 % eines mit wässrigen Medien schwer benetzbaren und/oder in wässrigen Medien schwer löslichen Antibiotikums führen.

Die erfindungsgemäß ausgerüsteten Darreichungsformen können daher insbesondere als Extrudate hergestellt werden, die durch Sphäronisierung in gerundete Pellets übergeführt werden können. Daher sind multipartikuläre durch Extrusion erhaltene Darreichungsformen, die Carrageenan und Tricalciumphosphat enthalten, wegen der möglichen, hohen Wirkstoffbeladung, der gegenüber Suspensionen geringeren Anforderung an eine Geschmacksneutralisierung und der raschen Auflösungsgeschwindigkeit bei einem pH-Wert von 6 -7 nach Auflösen etwaiger Überzüge besonders als erfindungsgemäße Darreichungsformen geeignet.

Vorzugsweise enthalten die erfindungsgemäßen Darreichungsformen 5 bis 30 Gew.%, bezogen auf das Gesamtgewicht der Darreichungsform, Carrageenan, vorzugsweise Kappa-Carrageenan.

Das Gewichtsverhältnis von Tricalciumphosphat zu Carrageenan beträgt in den erfindungsgemäßen Darreichungsformen, vorzugsweise 1:1 bis 1:10, besonders bevorzugt 1:2 bis 1:6.

Die erfindungsgemäß zusammengesetzten Darreichungsformen eignen sich bevorzugt für mit wässrigen Medien schwer benetzbare und/oder in wässrigen Medien schwer lösliche Antibiotika, deren Schwerlöslichkeit entsprechend der vorstehend aufgeführten Veröffentlichung "Guidance for Industry" definiert und klassifiziert ist. Vorzugsweise eignen sie sich für Antibiotika aus der Gruppe umfassend Penicilline, Cephalosporine und Macrolide, besonders bevorzugt für Amoxicillin, Clarithromycin, Azithromycin (mono- oder dihydrat), Cefixim, Cefpodoxim und/oder Cefpodoxim-Proxetil.

Neben der Kombination aus Carrageenan und Tricalciumphosphat kann die erfindungsgemäße Darreichungsform als Hilfsstoffe noch vorzugsweise wenigstens einen Sucroseester, vorzugsweise mit einem HLB Wert von 10-16, besonders bevorzugt von 13 - 15, enthalten. Weiterhin können die erfindungsgemäßen Darreichungsformen als Hilfsstoffe Füllmittel, Bindemittel, Gleitmittel, Farbstoffe oder Konservierungsstoffe aufweisen.

Die erfindungsgemäßen Darreichungsformen, wie Extrusionspellets, enthalten keine mikrokristalline Cellulose oder andere Sphäronisierungshilfsstoffe wie niedrig substituierte Hydroxypropylcellulose, Hydroxypropylmethylcellulose, Hydroxypropylcellulose, Pulvercellulose, Natrium-Carboxymethylcellulose, Polyvinylpyrrolidon, sofern sie noch nicht mit einem Überzug versehen wurden, d. h. in den nicht überzogenen Teilchen, wie Extrusionspellets, sind die genannten Hilfsstoffe nicht vorhanden.

In einer bevorzugten Ausführungsform sind die erfindungsgemäßen Darreichungsformen mit wenigstens einem Überzug, besonders bevorzugt mit einem Magensaft resistenten und/oder geschmacksneutralisierenden Überzug versehen, der vorzugsweise wieder über einen Schutzüberzug, der den Kern gegenüber dem Überzug isoliert, aufgetragen wird.

Diese Überzüge werden in einer Menge von 1 bis 50 Gew.%, bezogen auf das Gesamtgewicht der Darreichungsformen, je nach Art und Funktion des Überzuges aufgetragen.

Als Materiallen für einen Magensaft resistenten Überzug eignen sich vorzugsweise Methacrylsäure/Alkyl(meth)acrylat-Copolymere, besonders bevorzugt Copolymere von Methacrylsäure/Methylmethacrylat mit einem Verhältnis von 1:1 bis 1:2 wie Eudragit L^{®} oder Eudragit S^{®}, ganz besonders bevorzugt Copolymere von Methacrylsäure /Ethylacrylat 1:1 wie Eudragit L55^{®}, Eudragit L30D-55^{®}, die sich bei einem pH-Wert von ≥ 5,5 schnell auflösen. Weiterhin können als Magensaft resistente Überzüge Überzüge auf Basis von Cellulosen oder auf Basis von Schellack, die dem Fachmann bekannt sind, aufgetragen werden. Die Auftragung der Überzüge kann mit entsprechenden Lösungen oder Dispersionen in organischem oder wässrigem Medium erfolgen, wobei ein wässriges Medium bevorzugt ist. Als Speichel resistente Überzüge eignen sich vorzugsweise Überzüge auf Basis von Eudragit E oder Eudragit EP0.

Dem Fachmann ist es bekannt, dass den bekannten Überzugsmaterialien übliche Weichmacher, Farbstoffe, Gleitmittel, wie Talkum, Magnesiumstearat und/oder Glycerolmonostearat zugesetzt werden sollen bzw. können.

Erfindungsgemäß wird unter "Magensaft" sowohl die natürliche Komposition des Magensafts als auch die dem Fachmann bekannten künstlichen Magensaft ähnlichen Zubereitungen (pH 1,2 -2) verstanden. Ebenso werden unter "Freisetzung in Dünndarm" sowohl die Freisetzung im natürlichen Dünndarmsaft als auch die Freisetzung in Dünndarmsaft ähnlichen Zubereitungen bei pH-Werten von 6-7, vorzugsweise pH 6,4 - 6,8, wie sie in einschlägigen Arzneibüchern definiert sind, verstanden.

Die erfindungsgemäßen Darreichungsformen zeichnen sich dadurch aus, dass sie eine hohe Auflösungsgeschwindigkeit aufweisen und das Antibiotikum innerhalb von 30 min mit 85% freigesetzt wird, nachdem sich ein gegebenenfalls vorhandener Überzug vorher aufgelöst hat. Vorzugsweise erfolgt diese hohe Auflösungsgeschindigkeit bei pH-Werten von 6,4 - 6,8.

Die pH-abhängige Auflösungsdauer eines solchen Überzugs kann durch einfache Vorversuche in entsprechenden Standardpufferlösungen bestimmt werden.

Die erfindungsgemäßen Darreichungsformen werden hergestellt, indem man die Ausgangsstoffe mischt, granuliert, extrudiert, zerteilt und ggf. formt, vorzugsweise sphäronisiert, ggf. klassiert und ggf. mit einem Speichel und/oder Magensaft resistenten bzw. Geschmack neutralisierenden Überzug versieht.

Antibiotika, die oftmals eine bessere Löslichkeit bei niedrigen pH-Werten aufweisen, werden vorzugsweise mit Magensaft resistenten Überzügen geschützt, da dies die Geschmacksmaskierung bei Einnahme der Darreichungsform im Trinkhalm mit den bevorzugten Getränken, wie z. B. Cola oder Fruchtsäfte optimal unterstützt.

Dem Fachmann ist bekannt, daß die Komponenten gleichzeitig oder nacheinander der Mischung zugesetzt werden können. Ebenfalls kann die Mischung in einem bekannten Mischer oder Granulierer erfolgen, so dass gegebenenfalls die Mischung, die Granulierung und Extrusion gleichzeitig erfolgen können. Die Granulierung kann durch Feuchtgranulierung, vorzugsweise mit Wasser oder wässrigem Lösungsmittel erfolgen. Dem Fachmann sind geeignete Lösungsmittel bekannt.

Die Spharonisation, Extrusion und das Überziehen können jeweils in den dem Fachmann bekannten Apparaturen erfolgen. Für das Überziehen kann vorzugsweise eine Wirbelschicht-Apparatur eingesetzt werden.

In einer besonders bevorzugten, erfindungsgemäßen Darreichungsform liegt die multipartikuläre Darreichungsform in Form von sphärischen Extrusionspellets vor. Diese können vorzugsweise als Einmaldosis gemäß einem Darreichungssystem umfassend einen Trinkhalm mit vorzugsweise beweglichen Sperrvorrichtung, wie sie in WO 03/079957, WO 2004/000202, WO2004/000264 beschrieben ist, in diesen angeordnet sein und mit einer Transportflüssigkeit dem Patienten verabreicht werden.

Ein weiterer Gegenstand der vorliegenden Erfindung ist ein Darreichungssystem umfassend eine erfindungsgemäße Darreichungsform, vorzugsweise als Einmaldosis, angeordnet in einem Trinkhalm mit wenigstens einer vorzugsweise beweglichen Sperrvorrichtung zur Verabreichung mit Hilfe einer Transportflüssigkeit an einem menschlichen Patienten.

Als Transportflüssigkeiten eignen sich partikelfreie Getränke, vorzugsweise wässrige Flüssigkeiten, wie z. B. Wasser, vorzugsweise Mineralwasser, Tee, Fruchtsäfte, Coca-Cola, Limonaden, wobei Transportflüssigkeiten mit einem sauren pH-Wert bei Einsatz von Magensaft resistent überzogenen, multipartikulären Darreichungstormen, wie z. B. Extrusionspellets, bevorzugt sind.

Die Wirkstofffreisetzung der erfindungsgemäßen Darreichungsformen bzw. deren Auflösungsgeschwindigkeit wird nach der Methode bzw. Klassifizierung aus "Guidance for Industry, Waiver of In Vivo Bioavailability and Bioequivalence Studies for Immediate-Release Solid Oral Dosage Forms based on a Biopharmaceutics Classification System., Seiten 1-3 /7 , veröffentlicht vom U.S. Department of Health and Human Services , Food and Drug administration, Center for Drug Evaluation and Research (CDER), August 2000, BP" bestimmt.

Dazu wurde eine Freisetzungsapparatur mit Blattrührer gemäß U.S. Pharmacopeia eingesetzt und bei einer Temperatur von 37°C des Freisetzungsmediums und einer Umdrehungsgeschwindigkeit von 100 min-1 in der in den Beispielen angegebenen Zeit und dem dort angegebenen Freisetzungsmedium die Freisetzung gemessen. Die jeweils zu einem Zeitpunkt freigesetzte Menge des Wirkstoffes wurde durch HPLC oder UV-photometrisch bestimmt. Gemäß der Klassifizierung in der vorstehend genannten Veröffentlichung gilt eine unmittelbar freisetzende, feste Darreichungsform als eine Darreichungsform mit einer hohen Auflösungsgeschwindigkeit, wenn wenigstens 85% des in der Darreichungsform vorhandenen Wirkstoffs innerhalb von 15 bis 30 Minuten in einer vorgegebenen Pufferlösung mit einem bestimmten pH-Wert aufgelöst sind.

### Beispiele

### Unüberzogene Granulate

### Beispiel 1

### Extrusionspellets mit folgender Zusammensetzung

| Pro Dosis | Ausgangsstoffe |
|---|---|
| 250,0 mg | Clarithromycin Ph. Eur. |
| 100,0 mg | Carrageenan NF (Gelcarin GP-911, kappa-Carrageenan) |
| 50 mg | Tricalciumphosphat Ph. Eur. |
| 20,0 mg | Sucrosestearat S-1570, E473 |

wurden durch Mischung der Ausgangsstoffe in einem Schnellmischer und anschließender Feuchtgranulation und Extrusion des feuchten Granulats durch einen Extruder mit einer 0,5 x 0,5 mm Extrusionsmatrize bei Temperaturen des Extrudats unter 35°C hergestellt. In einem geeigneten Sphäroniser wurden die Extrudate sphäronisiert und die so erhaltenen Pellets in einem Wirbelschichttrockner bis zu einer Restfeuchte unter 10% getrocknet. Die getrockneten Pellets wurden mit Hilfe der Siebmethode klassiert und die 250 bis 710 µm Fraktion aller Siebungen vereinigt.

Von diesen nicht überzogenen Pellets wurde zunächst die Freisetzung in 900 ml Phosphatpufferlösung (pH 6,4) bei 37°C nach der vorstehend angegebenen Methode bei einer Umdrehungsgeschwindigkeit von 100 min⁻¹ für 60 min bestimmt. Die nachstehenden Werte zeigen das entsprechende Freisetzungsprofil von 3 parallelen Bestimmungen.

### Freisetzung von Clarithromycin

| | | | | | |
|---|---|---|---|---|---|
| Minuten | 0.0 | 15.0 | 30.0 | 45.0 | 60.0 |
| Clarithromycin | | | | | |
| T1 | 0.0 | 52.4 | 88,7 | 103.2 | 107.8 |
| T2 | 0.0 | 59.0 | 92.7 | 104.7 | 109.2 |
| T3 | 0.0 | 61.2 | 89.5 | 98.7 | 101.7 |

### Vergleichsbeispiel 1

Wie in Beispiel 1 angegeben, wurden Extrusionspellets folgender Zusammensetzung hergestellt:

| Pro Dosis | |
|---|---|
| 250,0 mg | Clarithromycin |
| 62,5 mg | Microcrystalline |
| | Cellulose |
| | (Avicell PH 105) |
| 50,0 mg | Niedrigsubstituierte |
| | Hydroxypropylcellulose |
| | NF |
| | (L-HPC, Typ LH 31) |
| 12,5 mg | Sucrosestearat (S-1570, E473) |

Die Freisetzung des Wirkstoffes aus den nicht überzogenen Pellets wurde nach der vorstehend angegeben Methode 900 ml Pufferlösung bei einem pH 6,4 bestimmt und nachstehend aufgeführt:

| | | | | | | | |
|---|---|---|---|---|---|---|---|
| Minuten | 10.0 | 15.0 | 30.0 | 45.0 | 60.0 | 90.0 | 120.0 |
| Clarithromycin (freigesetzt) | | | | | | | |
| T1 | 0.0 | 26.5 | 27.0 | 33.3 | 39.5 | 48.4 | 54.0 |
| T2 | 0.0 | 31.0 | 28.0 | 34.5 | 40.1 | 48.5 | 56.7 |
| T3 | 0.0 | 20.8 | 28.2 | 34.4 | 40.3 | 48.3 | 56.7 |

Die Werte zeigen, dass die mit microcrystalliner Cellulose durch Feuchtextrusion und Spheronisation hergestellten Pellets nicht zerfallen und nicht ausreichend schnell den Wirkstoff freisetzen.

### Vergleichsbeispiele 4 - 11

Wie in Beispiel 1 angegeben wurden Extrusionspellets mit 250 mg Clarithromycin und den in der Tabelle 1 angegebenen Hilfsstoffen hergestellt. Die Freisetzung von Clarithromycin in 900 ml Pufferlösung bei pH 6,8 und 150 Umdrehungen pro Minute nach der vorstehend angegebenen Methode ist ebenfalls in Tabelle 1 angegeben. Die Werte zeigen, dass weder durch den Einsatz von Carrageenan NF (Gelcarin GP-911) allein noch dessen Kombination mit weiteren Spheronisationshilfsstoffen Pellets erhalten werden, die ausreichend schnell zerfallen und ausreichend schnell den Wirkstoff freisetzten.

Nur bei Einsatz der erfindungsgemäßen Kombination von Carrageenan und Tricalciumphsophat entstehen Pellets mit der erforderlichen raschen Freisetzung für schwerlösliche bzw. schwer benetzbare Antibiotika.

**TABELLE 1**

| **Vergleichsbeispiel 4 - 11** | | | | | | |
|---|---|---|---|---|---|---|
| **Sucroseester** | **Gewicht pro Dosis** | **Spheronisations-Eigenschaften** | **Carrageenan pro Dosis** | **weitere Hilfsstoffe pro Dosis** | **Freisetzung einer 250 mg Dosis in 900 ml pH 6,8, 150 rpm** | |
| mg | mg | | mg | mg | % freigesetzt nach 15 min | % freigesetzt nach 30 min |
| 20 | 330 | Stränge, keine Kugel | 60 | 0 | 59 | 77 |
| 20 | 310 | Stränge, keine Kugel | 40 | 0 | 68 | 83 |
| 20 | 420 | gut, aber Pellets überwiegend > 700 µm | 100 | 50 Lactose x H₂O | 59 | 82 |
| 20 | 410 | Stränge, sehr langsamer Prozeß | 100 | 40 Mannitol | 55 | 79 |
| 20 | 380 | gut, aber Pellets überwiegend > 700 µm | 70 | 40 Mannitol | 52 | 69 |
| 20 | 420 | gut, aber Pellets überwiegend > 700 µm | 100 | 50 Mannitol | 60 | 79 |
| 20 | 420 | Agglomerationsneigung | 100 | 50 Calciumhydrogenphosphat | 52 | 75 |

### Beispiel 2

Wie in Beispiel 1 angegeben, wurden Extrusionspellets mit folgender Zusammensetzung hergestellt:

| Pro Dosis | Ausgangsstoffe |
|---|---|
| 447,6 mg | Cefixim x3H₂O, mikronisiert entsprechend |
| 400,0 mg | Cefixim USP |
| 50,6 mg | Tricalciumphosphat Ph. Eur. |
| 194,8 | Carrageenan NF |

Die Freisetzung des Wirkstoffes aus den nicht überzogenen Pellets wurde nach der vorstehend angegebenen Methode in 900 ml Pufferlösung bei einem pH-Wert 6,8 bestimmt und nachstehend aufgeführt:

| | | | | | |
|---|---|---|---|---|---|
| Minuten | 0.0 | 15.0 | 30.0 | 45.0 | 60.0 |
| Cefixim | | | | | |
| T1 | 0.0 | 86,6 | 94,0 | 94,2 | 94,2 |
| T2 | 0.0 | 86,4 | 93,9 | 93,7 | 93,7 |
| T3 | 0.0 | 87,1 | 93,8 | 94,1 | 94,1 |

### Beispiel 3

### Überzogene Granulate

Ein Teil der Pellets aus Beispiel 1 wurde jeweils mit wässrigen Dispersionen der folgenden Zusammensetzung überzogen:

### Dispersionszusammensetzung für die jeweiligen Überzüge

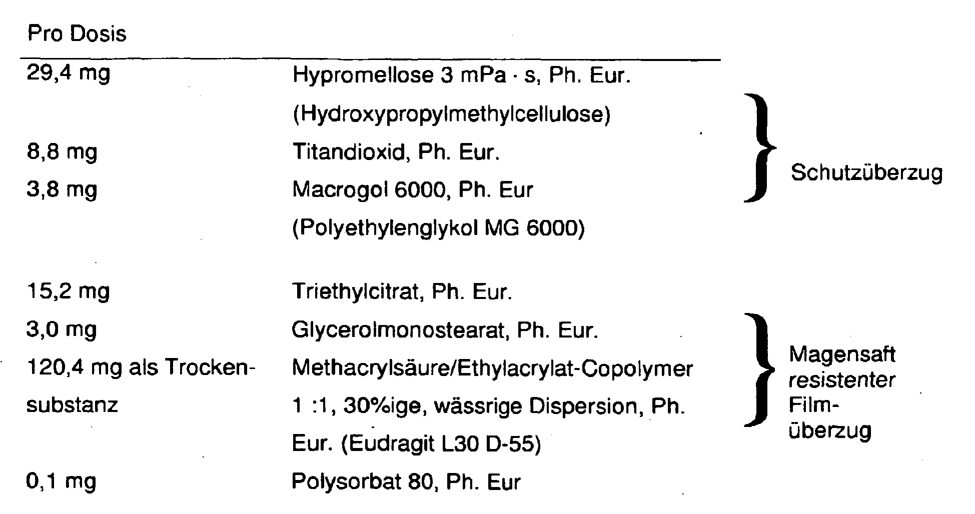

Zuerst wurden die Pellets mit einer wässrigen Dispersion mit 11 Gew.% Feststoffanteil und anschließend mit einer wässrigen Dispersion mit 15 Gew.% Feststoffanteil in einer Wirbelschichtanlage bei einer Produkttemperatur von 45°C bis zu einer Gewichtszunahme von 10 Gew.%, bezogen auf das Gewicht der Pellets, als Schutzüberzug und anschließend für den Erhalt eines Magensaft resistenten Filmüberzugs unter Warmluftzufuhr bei einer Produkttemperatur von 30°C bis zu einer Gewichtszunahme von 30 Gew%, bezogen auf das Gewicht der Pellets und des Schutzüberzugs, überzogen und unter verminderter Warmluftzufuhr bis zum Erreichen einer Produkttemperatur von 40°C und einer Restfeuchte von < 10% getrocknet.

Von den uberzogenen Pellets wurde nach der vorstehend angegebenen Methode bei einer Umdrehungsgeschwindigkeit von 100 min⁻¹ zunächst für 30 min. in 300 ml Pufferlösung bei pH 2 und anschließend für 60 min. in 1000 ml Phosphatpufferlösung bei pH 6,8 die Freisetzung des Wirkstoffes gemessen.

Es ergaben sich die nachstehenden Freisetzungen:

### Freisetzung der überzogenen Pellets

| | | | | | |
|---|---|---|---|---|---|
| Minuten | 0.0 - 30 | 45.0 | 60.0 | 75.0 | 90.0 |
| Clarithromycin | | | | | |
| T1 | 0.0 | 66.4 | 96.9 | 96.4 | 97.2 |
| T2 | 0.0 | 74.5 | 96.9 | 96.5 | 97.5 |
| T3 | 0.0 | 77.2 | 96.1 | 95.8 | 102.0 |
| T4 | 0.0 | 81.3 | 94.8 | 94.3 | 96.4 |
| T5 | 0.0 | 85.6 | 94.2 | 96.2 | 95.2 |
| T6 | 0.0 | 85.4 | 93.4 | 95.4 | 95.3 |

pH =2 für 30 min → pH = 6,8 für 60 min

### Vergleichsbeispiel 2

Wie in Beispiel 1 angegeben, wurden Extrusionspellets folgender Zusammensetzung hergestellt:

| Pro Dosis | |
|---|---|
| 250,0 mg | Clarithromycin |
| 75,0 mg | Microcrystalline Cellulose (PH 101, Ph. Eur.) |
| 25,0 mg | Niedrigsubstituierte |
| | Hydroxypropylcellulose NF (L-HPC, Typ LH2T) |
| 20,0 mg | Polysorbat 80 (Ph. Eur.) |
| 180,0 mg | Lactose-Monohydrat (Typ. 230, Ph. Eur.) |
| 550,0 mg | Gereinigtes Wasser (Ph. Eur.) |

Die so erhaltenen Extrusionspellets wurden mit einer wässrigen Dispersion folgender Zusammensetzung überzogen:

### Zusammensetzung der wässrigen Dispersion zum Überziehen

| Pro Dosis | |
|---|---|
| 69,5 mg | Talkum (Ph. Eur.) |
| 13,2 mg | Triethylcitrat (Ph. Eur.) |
| 66,0 mg als Trockensubstanz | Methacrylsäure/Ethylacrylat Copolymer 1:1, 30%ige wässrige Dispersion, Ph. Eur. (Eudragit L30 D55) |

Die Gewichtszunahme, bezogen auf das Gewicht der Pellets, durch den Überzug betrug 18 Gew.%.

Von einem Teil der überzogenen Pellets wurde die Freisetzung des Wirkstoffes nach der vorstehend beschriebenen Methode in 900 ml Pufferlösung bei pH 6,4 bestimmt und nachstehend angegeben:

| | | | | | |
|---|---|---|---|---|---|
| Minuten | 0.0 | 15.0 | 30.0 | 45.0 | 60.0 |
| Clarithromycin | | | | | |
| T1 | 0.0 | 44.6 | 71.3 | 84.2 | 92.3 |
| T2 | 0.0 | 50.2 | 75.8 | 89.9 | 97.2 |

Trotz des schnellen Auflösen des Überzugs bei pH 6,4 erfolgt die Freisetzung aus den Pellets nur sehr langsam, da kein Zerfall der Pellets zu beobachten ist.

Von einem Teil der überzogenen Pellets wurde die Freisetzung des Wirkstoffes zunächst in 300 ml bei pH 2 für 30 min und anschließend in 100 ml bei pH 6,8 nach der vorstehend angegebenen Methode bestimmt und nachstehend angegeben:

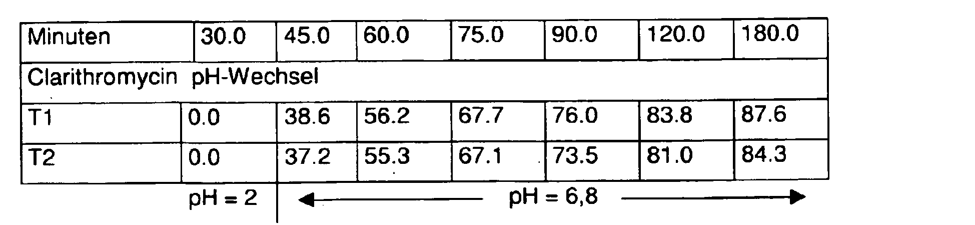

Die Pellets quollen in Magensaft, obwohl sich der Überzug bei pH 2 nicht löste. Er war aber für Wasser permeabel. Dennoch kam es selbst nach dessen vollständiger Auflösung bei pH 6,8 zu einer gegenüber den unüberzogenen Pellets deutlich verzögerten Freisetzung und keinem Zerfall der Pellets.

### Beispiel 4

Wie in Beispiel 1 angegeben, wurden Extrusionspellets mit folgender Zusammensetzung hergestellt:

| pro Dosis | Ausgangsstoffe |
|---|---|
| 575,00 mg | Amoxicillin-Trihydrat Ph. Eur., = 500 mg Amoxicillin, wasserfrei |
| 65,00 mg | Tricalciumphosphat, Ph. Eur. |
| 250,00 mg | Carrageenan NF |

Von den vereinigten Siebfraktionen mit einer Teilchengröße von 250 bis 710 µm wurde die Freisetzung des Wirkstoffes aus den nicht überzogenen Pellets in Phosphatpuffer (pH 6,8) in 900 ml für 30 Minuten nach der vorstehend angegebenen Methode bestimmt und in der nachfolgenden Tabelle angegeben:

| | | | | | | |
|---|---|---|---|---|---|---|
| Minuten | 0.0 | 1.0 | 15.0 | 10.0 | 15.0 | 30.0 |
| Amoxicillin | | | | | | |
| T1 | 0.0 | 23.9 | 97.8 | 100.7 | 100.6 | 97.5 |
| T2 | 0.0 | 53.9 | 107.1 | 104.8 | 101.7 | 101.0 |
| T3 | 0.0 | 46.6 | 99.4 | 93.6 | 91.2 | 90.0 |

Wie in Beispiel 3 angegeben, wurde ein Teil der Extrusionspellets auch mit einer wässrigen Dispersion mit der folgender Zusammensetzung bis zu einer Gewichtszunahme von 2 Gew.%, bezogen auf das Gewicht der Pellets, überzogen:

### Zusammensetzung der wässrigen Überzugsdispersionen

| pro Dosis | |
|---|---|
| 15,51 mg als Trockensubstanz | Methacrylsäure/Ethylacrylat Copolymer 1:1, 30%ige wässrige Dispersion, (Eudragit L30 D-55), Ph. Eur. |
| 1,91 mg | Triethylcitrat, Ph. Eur. |
| 0,36 mg | Glycerinmonostearat (Cutina V, pflanzlich), Ph. Eur. |
| 0,02 mg | Polysorbat 80 Ph. Eur. |

Die Bestimmung der Freisetzung nach der vorstehend beschriebenen Methode bei pH 6,8 in 900 ml Pufferlösung für 10 Minuten ergab folgende Werte:

| | | | |
|---|---|---|---|
| Minuten | 0.0 | 5.0 | 10.0 |
| Amoxicillin (freigesetzt) | | | |
| T1 | 0.0 | 86.2 | 96.9 |
| T2 | 0.0 | 85.1 | 98.0 |
| T3 | 0.0 | 85.3 | 97.1 |

Die Freisetzungswerte zeigten für überzogene und nicht überzogene Amoxcillin-Pellets nahezu gleich verlaufende Freisetzungsprofile. Dies verdeutlicht, dass nicht der Überzug, der lediglich zur Geschmacksmaskierung dient, sondern die erfindungsgemäße Zusammensetzung der Darreichungsform eine sehr schnelle Freisetzung im Dünndarmmilieu und damit eine rasche Bioverfügbarkeit ermöglicht.

### Vergleichsbeispiel 3

Wie in Beispiel 1 angegeben, wurden Extrusionspellets mit folgender Zusammensetzung hergestellt:

| pro Dosis | Ausgangsstoffe |
|---|---|
| 581,00 mg | Amoxicillin-Trihydrat = 500 mg |
| | Amoxicillin, wasserfrei, Ph. Eur. |
| 73,0 mg | Mikrokristalline Cellulose, Typ PH101, |
| | Ph. Eur. |
| 73,00 mg | Niedrigsubstituierte |
| | Hydroxypropyolcellulose, Typ LH21, NF |

Von diesen Extrusionspellets wurde die Freisetzung des Wirkstoffes nach der vorstehend angegebenen Methode in 900 ml Pufferlösung bei pH 6,8 bestimmt und nachfolgend angegeben:

| | | | | | |
|---|---|---|---|---|---|
| Minuten | 0.0 | 15.0 | 30.0 | 45.0 | 60.0 |
| Amoxicillin (freigesetzt) | | | | | |
| T1 | 0.0 | 29.2 | 43.4 | 59.5 | 69.7 |
| T2 | -0.1 | 29.8 | 44.2 | 60.8 | 71.0 |
| T3 | 0.0 | 29.3 | 43.9 | 60.0 | 70.4 |
| T4 | 0.1 | 29.4 | 43.8 | 60.2 | 70.5 |
| T5 | 0.0 | 29.4 | 43.7 | 59.8 | 70.2 |
| T6 | -0.1 | 29.3 | 43.2 | 59.6 | 69.7 |

Wie in Beispiel 3 angegeben, wurde ein Teil der Extrusionspellets auch mit einer wässrigen Dispersion mit der folgender Zusammensetzung bis zu einer Gewichtszunahme von 2 Gew.%, bezogen auf das Gewicht der Pellets, überzogen:

### Zusammensetzung der wässrigen Überzugsdispersionen

| pro Dosis | |
|---|---|
| 15,51 mg als Trockensubstanz | Methacrylsäure/Ethylacrylat Copolymer 1:1, 30%ige wässrige Dispersion, |
| | (Eudragit L30 D-55), Ph. Eur. |
| 1,91 mg | Triethylcitrat, Ph. Eur. |
| 0,36 mg | Glycerolmonostearat (Cutina V, pflanzlich), Ph. Eur. |
| 0,02 mg | Polysorbat 80 Ph. Eur. |

Die Bestimmung der Freisetzung nach der vorstehend beschriebenen Methode bei pH 1,2 in 900 ml Pufferlösung für 90 Minuten ergab folgende Werte:

| | | | | | | | |
|---|---|---|---|---|---|---|---|
| Minuten | 0.0 | 6.0 | 15.0 | 30.0 | 45.0 | 60.0 | 90.0 |
| Amoxicillin | | | | | | | |
| Mittelwert aus n=3 | 0 | 8 | 20 | 35 | 43 | 50 | 58 |

### Beispiel 5

Wie in Beispiel 1 angegeben, wurden Extrusionspellets mit folgender Zusammensetzung hergestellt:

| pro Dosis | |
|---|---|
| 20.0 mg | Succrosestearat |
| | S-1570 |
| 250.0 mg = | Azithromycin |
| 256 mg | (Monohydrat) |
| 50.0 mg | Tricalciumphosphat |
| 100.0mg | Carrageenan NF |

Die vereinigten Siebfraktionen mit einer Teilchengröße von 250 bis 710 µm wurden mit den nachstehend beschriebenen Überzugsdispersionen, wie in Beispiel 3 angegeben, überzogen:

### Zusammensetzung der wässrigen Überzugsdispersion

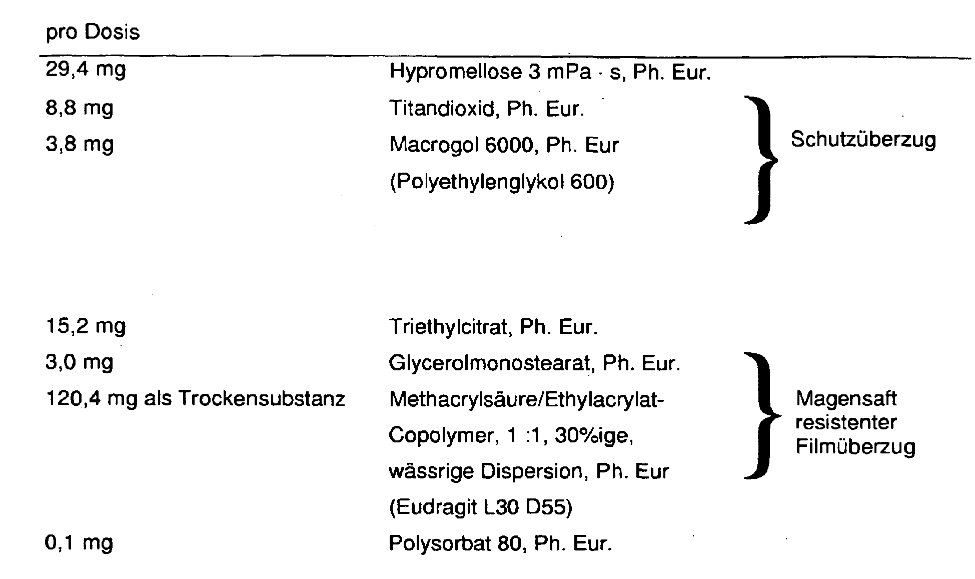

Von den überzogenen Pellets wurde die Wirkstofffreisetzung in 900 ml Phosphatpufferlösung (pH 6,4) bei 37°C nach der vorstehend angegeben Methode gemessen und nachfolgend aufgeführt:

| | | | | |
|---|---|---|---|---|
| Minuten | 0.0 | 15.0 | 30.0 | 45.0 |
| Freisetzung Azithromycin | | | | |
| T1 | 0.0 | 96.3 | 96.5 | 95.7 |
| T2 | 0.0 | 95.0 | 94.4 | 93.5 |
| T3 | 0.0 | 94.8 | 94.1 | 93.1 |

## Patentansprüche

1. Multipartikuläre, pharmazeutische Darreichungsform enthaltend wenigstens ein mit wässrigen Medien schwer benetzbares und/oder in wässrigen Medien schwer lösliches Antibiotikum und eine Kombination aus Carrageenan und Tricalciumphosphat und ggf. wenigstens einem Sucroseester.

2. Darreichungsform nach Anspruch 1, **dadurch gekennzeichnet, dass** sie in Form von Extrusionspellets, vorzugsweise in sphärischer Form, vorliegt.

3. Darreichungsform nach Anspruch 1 oder 2, **dadurch gekennzeichnet, dass** der Sucroeester einen HLB von 10-16, vorzugsweise 13 - 15 aufweist.

4. Darreichungsform nach einem der Ansprüche 1 bis 3, **dadurch gekennzeichnet, dass** sie 5 bis 50 Gew.%, vorzugsweise 20 - 30 Gew% bezogen auf das Gesamtgewicht der Darreichungsform, Carrageenan, vorzugsweise kappa-Carrageenan, enthält.

5. Darreichungsform nach einem der Ansprüche 1 bis 4, **dadurch gekennzeichnet, dass** das Gewichtsverhältnis von Tricalciumphosphat zu Carrageenan 1:1 bis 1:10, vorzugsweise 1:2 bis 1:6, beträgt.

6. Darreichungsform nach einem der Ansprüche 1 bis 5, **dadurch gekennzeichnet, dass** das Tricalciumphosphat als fein verteiltes Pulver mit einer Teilchengröße < 50 µm vorliegt.

7. Darreichungsform nach einem der Ansprüche 1 bis 6, **dadurch gekennzeichnet, dass** der Sucroseester in Mengen von 1-10 Gew%, vorzugsweise 4-6 Gew%, bezogen auf das Gesamtgewicht der Darreichungsform, vorliegt.

8. Darreichungsform nach einem der Ansprüche 1 bis 7, **dadurch gekennzeichnet, dass** sie wenigstens ein Antibiotikum ausgewählt aus der Gruppe bestehend aus Penicillinen, Cephalosporinen und Macroliden enthält.

9. Darreichungsform nach Anspruch 8, **dadurch gekennzeichnet, dass** das Antibiotikum Amoxicillin, Clarithromycin, Azithromycin (Mono- oder Dihydrat) oder Cefixim Cefpodoxim und/oder Cefpodoxim-Proexetil ist.

10. Darreichungsform nach einem der Ansprüche 1 bis 9, **dadurch gekennzeichnet, dass** die Darreichungsform einen Magensaft resistenten und/oder Speichel resistenten als geschmacksneutralen Überzug aufweist.

11. Darreichungsform nach einem der Ansprüche 1 bis 10, **dadurch gekennzeichnet**, das die Partikel eine Größe < 800 µm aufweisen.

12. Darreichungsform nach einem der Ansprüche 1 bis 11, **dadurch gekennzeichnet, dass** sie eine hohe Auflösungsgeschwindigkeit bei eine pH-Wert von 6-7 aufweist.

13. Darreichungsform nach Anspruch 12, **dadurch gekennzeichnet, dass** das Antibiotikum innerhalb von 30 Min. in einer Menge von wenigstens 85% bei einem pH-Wert von 6 - 7 freigesetzt wird.

14. Darreichungsform nach einem der Ansprüche 1 bis 13, **dadurch gekennzeichnet, dass** die Darreichungsform Amoxicillin enthält und einen geschmacksneutralisierenden Überzug aufweist.

15. Darreichungsform nach einem der Ansprüche 1 bis 13, **dadurch gekennzeichnet, dass** die Darreichungsform Clarithromycin enthält und einen geschmacksneutralsierenden Überzug aufweist.

16. Verfahren zur Herstellung einer Darreichungsform nach einem der Ansprüche 1 bis 15, **dadurch gekennzeichnet, dass** man die Ausgangsstoffe mischt, granuliert, extrudiert, zerteilt, spharonisiert, ggf. klassiert und ggf. mit einem Speichel resistenten und/oder Magensaft resistenten und/oder geschmacksneutralen Überzug versieht.

17. Verfahren nach Anspruch 16, **dadurch gekennzeichnet, dass** man die Ausgangsmischung feucht granuliert.

18. Darreichungssystem umfassend eine Darreichungsform in Form von vorzugsweise gerundeten Extrusionspellets nach einem der Ansprüche 1 bis 15 angeordnet in einem Trinkhalm mit wenigstens einer vorzugsweise beweglichen Sperrvorrichtung zur einmaligen Verabreichung.

19. Kit aus einem Darreichungssystem gemäß Anspruch 18 und einer physiologisch verträglichen Transportflüssigkeit mit einem pH ≤ 6.

## Claims

1. A multiparticulate, pharmaceutical dosage form containing at least one antibiotic which is sparingly wettable with aqueous media and/or sparingly soluble in aqueous media and a combination of carrageenan and tricalcium phosphate and optionally at least one sucrose ester.

2. A dosage form according to claim 1, **characterised in that** it assumes the form of extruded pellets, preferably in spherical form.

3. A dosage form according to claim 1 or claim 2, **characterised in that** the sucrose ester exhibits an HLB of 10-16, preferably of 13-15.

4. A dosage form according to any one of claims 1 to 3, **characterised in that** it contains 5 to 50 wt.%, preferably 20-30 wt.%, relative to the total weight of the dosage form, of carrageenan, preferably kappa-carrageenan.

5. A dosage form according to any one of claims 1 to 4, **characterised in that** the weight ratio of tricalcium phosphate to carrageenan amounts to 1:1 to 1:10, preferably 1:2 to 1:6.

6. A dosage form according to any one of claims 1 to 5, **characterised in that** the tricalcium phosphate is present as a finely divided powder with a particle size of < 50 µm.

7. A dosage form according to any one of claims 1 to 6, **characterised in that** the sucrose ester is present in quantities of 1-10 wt.%, preferably of 4-6 wt.%, relative to the total weight of the dosage form.

8. A dosage form according to any one of claims 1 to 7, **characterised in that** it contains at least one antibiotic selected from the group consisting of penicillins, cephalosporins and macrolides.

9. A dosage form according to claim 8, **characterised in that** the antibiotic is amoxicillin, clarithromycin, azithromycin (mono- or dihydrate) or cefixim, cefpodoxime and/or cefpodoxime proxetil.

10. A dosage form according to any one of claims 1 to 9, **characterised in that** the dosage form comprises a gastric juice-resistant and/or saliva-resistant, flavour-neutral coating.

11. A dosage form according to any one of claims 1 to 10, **characterised in that** the particles have a size of < 800 µm.

12. A dosage form according to any one of claims 1 to 11, **characterised in that** it exhibits an elevated dissolution rate at a pH value of 6-7.

13. A dosage form according to claim 12, **characterised in that**, at a pH value of 6-7, the antibiotic is released in a quantity of at least 85% within 30 min.

14. A dosage form according to any one of claims 1 to 13, **characterised in that** the dosage form contains amoxicillin and comprises a flavour-neutralising coating.

15. A dosage form according to any one of claims 1 to 13, **characterised in that** the dosage form contains clarithromycin and comprises a flavour-neutralising coating.

16. A method for producing a dosage form according to any one of claims 1 to 15, **characterised in that** the starting materials are mixed, granulated, extruded, subdivided, spheronised, optionally classified and optionally provided with a saliva-resistant and/or gastric juice-resistant and/or flavour-neutral coating.

17. A method according to claim 16, **characterised in that** the starting mixture is moist granulated.

18. An administration system comprising a dosage form in the form of preferably rounded extruded pellets according to any one of claims 1 to 15 arranged in a drinking straw with at least one preferably mobile barrier device for single administration.

19. A kit comprising an administration system according to claim 18 and a physiologically acceptable conveying liquid with a pH of ≤ 6.

## Revendications

1. Forme d'administration pharmaceutique, multiparticulaire, contenant au moins un antibiotique difficilement mouillable par des milieux aqueux et/ou peu soluble dans des milieux aqueux et une combinaison de carraghénane et de phosphate tricalcique et le cas échéant au moins un ester de saccharose.

2. Forme d'administration selon la revendication 1, **caractérisée en ce qu'**elle se trouve sous forme de pellets extrudés, de préférence sous forme sphérique.

3. Forme d'administration selon la revendication 1 ou 2, **caractérisée en ce que** l'ester de saccharose présente une BHL de 10-16, de préférence de 13-15.

4. Forme d'administration selon l'une quelconque des revendications 1 à 3, **caractérisée en ce qu'**elle contient 5 à 50% en poids, de préférence 20-30% en poids, par rapport au poids total de la forme d'administration, de carraghénane, de préférence de carraghénane kappa.

5. Forme d'administration selon l'une quelconque des revendications 1 à 4, **caractérisée en ce que** le rapport pondéral de phosphate tricalcique à carraghénane est de 1:1 à 1:10, de préférence de 1:2 à 1:6.

6. Forme d'administration selon l'une quelconque des revendications 1 à 5, **caractérisée en ce que** le phosphate tricalcique est trouve sous forme de poudre finement répartie présentant une grosseur de particules < 50 µm.

7. Forme d'administration selon l'une quelconque des revendications 1 à 6, **caractérisée en ce que** l'ester de saccharose se trouve en des quantités de 1 à 10% en poids, de préférence de 4-6% en poids, par rapport au poids total de la forme d'administration.

8. Forme d'administration selon l'une quelconque des revendications 1 à 7, **caractérisée en ce qu'**elle contient au moins un antibiotique choisi dans le groupe constitué par les pénicillines, les céphalosporines et les macrolides.

9. Forme d'administration selon la revendication 8, **caractérisée en ce que** l'antibiotique est l'amoxicilline, la clarithromycine, l'azithromycine (monohydratée ou dihydratée) ou le céfixime, le cefpodoxime et/ou le cefpodoxime-proxétil.

10. Forme d'administration selon l'une quelconque des revendications 1 à 9, **caractérisée en ce que** la forme d'administration présente un revêtement résistant au suc gastrique et/ou à la salive comme revêtement de goût neutre.

11. Forme d'administration selon l'une quelconque des revendications 1 à 10, **caractérisée en ce que** les particules présentent une grosseur < 800 µm.

12. Forme d'administration selon l'une quelconque des revendications 1 à 11, **caractérisée en ce qu'**elle présente une vitesse de dissolution élevée à un pH de 6-7.

13. Forme d'administration selon la revendication 12, **caractérisée en ce que** l'antibiotique est libéré en 30 min en une quantité d'au moins 85% à un pH de 6-7.

14. Forme d'administration selon l'une quelconque des revendications 1 à 13, **caractérisée en ce que** la forme d'administration contient de l'amoxicilline et présente un revêtement neutralisant le goût.

15. Forme d'administration selon l'une quelconque des revendications 1 à 13, **caractérisée en ce que** la forme d'administration contient de la clarithromycine et présente un revêtement neutralisant le goût.

16. Procédé pour la préparation d'une forme d'administration selon l'une quelconque des revendications 1 à 15, **caractérisé en ce qu'**on mélange les substances de départ, on les granule, on les extrude, on les réduit, on les sphéronise, on le classe le cas échéant et on les pourvoit le cas échéant d'un revêtement résistant à la salive et/ou résistant au suc gastrique et/ou de goût neutre.

17. Procédé selon la revendication 16, **caractérisé en ce qu'**on granule le mélange de départ à l'état humide.

18. Système d'administration comprenant une forme d'administration sous forme de pellets d'extrusion de préférence arrondis selon l'une quelconque des revendications 1 à 15 disposés dans une paille avec au moins un dispositif de blocage de préférence mobile pour une administration unique.

19. Kit formé par un système d'administration selon la revendication 18 et un liquide de transport physiologiquement compatible présentant un pH ≤ 6.
